Europäisches Patentamt

European Patent Office

Office européen des brevets

(19)

(11) Veröffentlichungsnummer: **0 242 711**
**B1**

(12) **EUROPÄISCHE PATENTSCHRIFT**

(45) Veröffentlichungstag der Patentschrift:
26.09.90

(51) Int. Cl.⁵: **A61B 17/43**

(21) Anmeldenummer: 87105195.9

(22) Anmeldetag: 08.04.87

(54) **Katheter zur künstlichen Befruchtung.**

(30) Priorität: 09.04.86 AT 921/86

(43) Veröffentlichungstag der Anmeldung:
28.10.87 Patentblatt 87/44

(45) Bekanntmachung des Hinweises auf die Patenterteilung:
26.09.90 Patentblatt 90/39

(84) Benannte Vertragsstaaten:
BE CH DE ES FR GB GR IT LI LU NL SE

(56) Entgegenhaltungen:
EP-A- 0 122 571
DE-A- 3 315 934
FR-A- 2 556 596
US-A- 3 777 743
US-A- 4 560 376

(73) Patentinhaber: Fischl, Franz H., Dr., Weimarer
Strasse 5/16, A-1180 Wien(AT)
Patentinhaber: Pickhard, Ewald, Redtenbachergasse 15,
A-1160 Wien(AT)

(72) Erfinder: Fischl, Franz H., Dr., Weimarer Strasse 5/16,
A-1180 Wien(AT)
Erfinder: Pickhard, Ewald, Redtenbachergasse 15,
A-1160 Wien(AT)

(74) Vertreter: Wolke, Heidemarie, Dr., Stadtplatz 7,
A-4400 Steyr(AT)

## Beschreibung

Die Erfindung betrifft einen Katheter zur künstlichen Befruchtung mit einem in die Gebärmutter bzw. durch den inneren Muttermund in das Uteruskavum einführbaren flexiblen schlauchförmigen Katheterteil, der in einem, einem Zervixkanal zugeordneten Endbereich einen sich daran anschließenden flexiblen schlauchförmigen Fortsatz aufweist, dessen Länge im wesentlichen der Länge einer Scheide entspricht und im anderen Endbereich stirnseitig verschlossen und abgerundet bzw. kugelkalottenförmig ausgebildet und mit einem Kanal, der vor dem abgerundeten Endbereich mit radial angeordneten Ausströmöffnungen verbunden ist, wobei zwischen dem Katheterteil und dem Fortsatz ein sich in Richtung des Fortsatzes vergrößernder kegelförmiger Ansatz angeordnet ist. Solch ein Katheter ist aus US-A 3 777 743 bekannt.

Zudem sind Katheter zur künstlichen Befruchtung bekannt gemäß EP-A 1 122 571, die sich von dem der Scheide zugewendeten Ende des Zervixkanals in das Uteruskavum erstrecken. Im Eingangsbereich des Zervixkanals ist eine Kupplungsvorrichtung vorgesehen, mit der der Katheterkanal mit einem Auslaß einer Spritze verbunden wird. Die Spritze befindet sich innerhalb der Scheide und wird über eine Hebelanordnung von außerhalb der Scheide betätigt, wozu in die Scheide ein Stützapparat eingesetzt wird. Diese Art der künstlichen Befruchtung hat sich jedoch nicht sehr bewährt, weil die Handhabung dieser verschiedenen Teile relativ kompliziert ist und auch durch die Vielzahl der benötigten Einzelteile die zum Einbringen des Samens benötigten Gerätschaften nicht völlig steril gehalten werden können, was die Infektionsgefahr in der Scheide und Gebärmutter erhöhen und damit den Erfolg der Insemination beeinträchtigen kann.

Der vorliegenden Erfindung liegt die Aufgabe zugrunde, einen Katheter zu schaffen, mit dem eine für die Patientin schmerzlose, sterile und verletzungsfreie künstliche Befruchtung mittels Insemination möglich ist.

Diese Aufgabe der Erfindung wird dadurch gelöst, daß der Kanal des schlauchförmigen Katheterteiles in dem den kegelförmigen Ansatz gegenüberliegenden Endbereich mit nur zwei in Umfangsrichtung versetzten, radial verlaufenden Ausströmöffnungen versehen ist, von welchen die eine Ausströmöffnung mit einem großen Querschnitt und die weitere Ausströmöffnung mit einem geringeren Ausströmquerschnitt als die eine Ausströmöffnung ausgebildet oder durch einen luftdurchlässigen, insbesondere der einen Ausströmöffnung gegenüberliegenden Bereich im Schlauchmantel des schlauchförmigen Katheterteiles gebildet ist. Der überraschende Vorteil dieser scheinbar einfachen Lösung liegt nunmehr darin, daß sich der Katheter vom Scheideneingang bis in das Uteruskavum erstreckt und somit eine einwandfreie Samenzufuhr in das Uteruskavum möglich ist. Die flexible Ausgestaltung ermöglicht es, trotz der nötigen Festigkeit für das Einbringen in den Zervikalkanal, daß sich dieser der jeweiligen Führung des Zervikalkanals auch bei starker avfl.-oder rvfl.-Lage des Uterus problemlos anpassen kann. Damit ist ein schonendes Einführen durch den inneren Muttermund in das Uteruskavum ohne Fixation der Zervix möglich. Es wird aber auch eine Verletzung der Schleimhaut in diesem Bereich und somit eine mögliche Blutung verhindert und damit eine Störung der Befruchtung durch diese Blutungen ausgeschaltet. Ein weiterer wesentlicher Vorteil der erfindungsgemäßen Lösung liegt aber auch darin, daß dieser einstückige sterile Teil im Uteruskavum mit zwei Ausströmöffnungen versehen ist, wodurch eine Reizung bzw. Irritation oder sogar Verletzung der Schleimhaut im Uteruskavum durch das Austreten von komprimierter Luft, wie dies beim Einspritzen von Nativsamen oder in verschiedenen Nährmedien aufbereitenden Samen durch die darin enthaltenen Luftblasen möglich ist, verhindert wird. Dadurch werden die bisher aufgetretenen, unkontrollierbaren Verletzungen der Schleimhaut, die mit Blutaustritt und somit einer Einschränkung der Samenqualität verbunden waren, verhindert, und die Erfolgsrate bei der künstlichen Befruchtung wird dadurch verbessert.

Nach einer weiteren Ausführungsform ist vorgesehen, daß der Kanal des schlauchförmigen Fortsatzes in dem vom kegelförmigen Ansatz abgewendeten Endbereich– wie an sich bekannt– konusförmig erweitert, insbesondere als Luer-Konus ausgebildet ist. Damit ist es möglich, den Katheterkanal über den schlauchförmigen Fortsatz unmittelbar mit einer Spritze zu verbinden, was insbesondere dadurch erleichtert wird, wenn der Kanal in seinem aus der Scheide herausragenden Endbereich mit einem Luer-Konus ausgebildet ist.

Nach einer anderen Weiterbildung ist es möglich, daß zwischen dem kegelförmigen Ansatz und dem schlauchförmigen Fortsatz ein coaxial zur Bohrung verlaufender kreisringförmiger, insbesondere kegelstumpfartiger Wulst angeordnet ist. Dieser kreisringförmige, insbesondere kegelstumpfförmige Wulst ermöglicht eine bessere Abstützung des Katheters am Eingang des Zervixkanals, sodaß auch während der künstlichen Befruchtung ein zu tiefes Einschieben des Katheters in das Uteruskavum verhindert wird.

Es ist aber auch möglich, daß der Fortsatz und der schlauchförmige Katheterteil über eine Verbindungsvorrichtung lösbar verbunden sind, dadurch können einfach unterschiedlich lange Teile als Katheter verwendet werden.

Von Vorteil ist es weiters aber auch, wenn der Katheter einstückig ausgebildet und insbesondere aus einem elastischen Kunststoff, z.B. Polyäthylen bzw. Polypropylen oder Weich-PVC besteht, da dadurch während des Einspritzens des Samens eine durchgehende dichte Verbindung vom Scheideneingang bis in das Uteruskavum geschaffen ist und somit der gesamte Samen mit hoher Sicherheit in den gewünschten Bereich des Uteruskavum gebracht wird.

Nach einer weiteren Ausbildung der Erfindung ist vorgesehen, daß die eine Ausströmöffnung im wesentlichen rechteckförmig ausgebildet ist und eine kürzere Seitenlänge derselben im wesentlichen einem Durchmesser des Kanales entspricht, da dadurch ein zu hoher Druck beim Austreten des Samens aus dem Kanal des Katheters verhindert wird.

Weiters ist es aber auch von Vorteil, wenn die weitere Ausströmöffnung der einen Ausströmöffnung spiegelbildlich gegenüberliegt, da dadurch der Druck von im Nativsamen bzw. in Nährlösungen enthaltener Samen während des Einspritzens enthaltenen komprimierten Luft bevor der Samen zur Gänze aus dem Kanal des Katheters ausgetreten ist, über die gegenüberliegende Entlastungsbohrung verringert und somit ein Druckstoß durch zu stark verdichtete Luft und eine damit einhergehende Irritation der Schleimhaut verhindert wird.

Schließlich ist es auch möglich, daß der Durchmesser des schlauchförmigen Katheterteiles in etwa halb so groß ist wie der Durchmesser des schlauchförmigen Fortsatzes, wodurch das Einführen des sich von der Ausströmöffnung bis in den Bereich des konusförmigen Ansatzes erstreckenden Bereiches des Katheters in das Uteruskavum erleichtert wird.

Zum besseren Verständnis der Erfindung wird diese im folgenden anhand der in den Zeichnungen dargestellten Ausführungsbeispiele eines Katheters näher erläutert.

Es zeigen:

Fig. 1 einen erfindungsgemäß ausgebildeten Katheter mit angesetzter Spritze;
Fig. 2 einen Schnitt durch den Katheter in einem den Ausströmöffnungen zugeordneten Endbereich gemäß den Linien II–II in Fig. 1;
Fig. 3 einen Schnitt durch den Katheter in einem den Ausströmöffnungen zugeordneten Endbereich gemäß den Linien III–III in Fig. 1 sowie eine mögliche Verbindungsvorrichtung zwischen dem schlauchförmigen Katheterteil und dem schlauchförmigen Fortsatz;
Fig. 4 den erfindungsgemäß ausgebildeten Katheter bei in das Uteruskavum eingeführter Stellung mit der Gebärmutter in Seitenansicht und geschnitten;
Fig. 5 den erfindungsgemäß ausgebildeten Katheter bei in das Uteruskavum eingeführter Stellung mit der Gebärmutter in Stirnansicht und geschnitten.

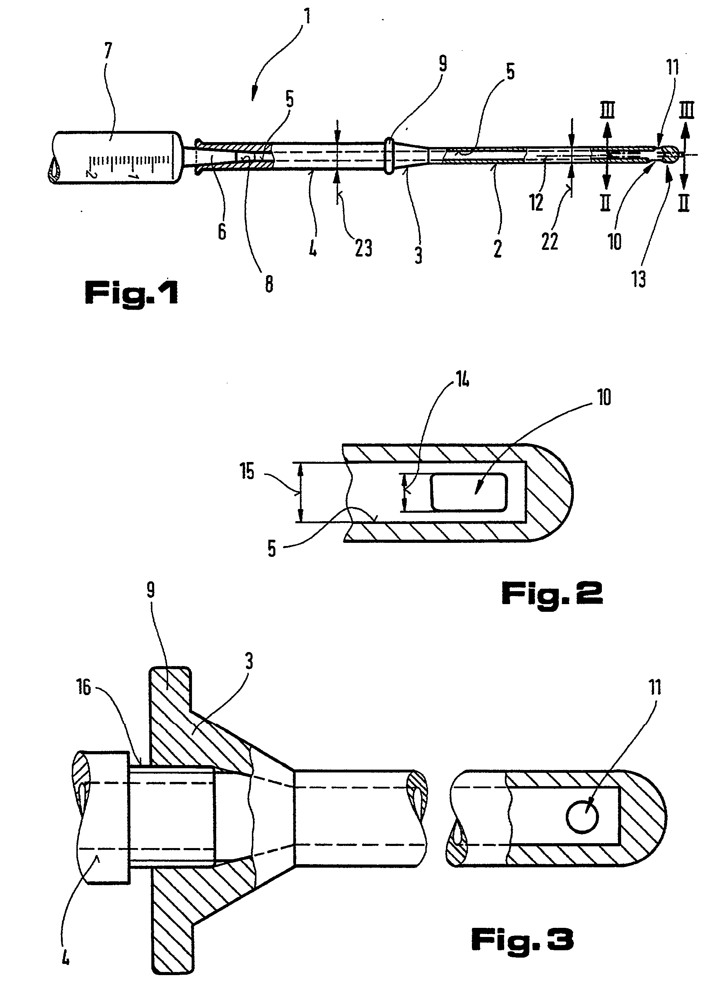

In Fig.1 ist ein Katheter 1 zur künstlichen Befruchtung gezeigt, der einen flexiblen schlauchförmigen Katheterteil 2 einen an diesen anschließenden, im vorliegenden Ausführungsbeispiel kegelförmigen Ansatz 3 und einen sich vom kegelförmigen Ansatz 3 in entgegengesetzter Richtung zum schlauchförmigen Katheterteil 2 erstreckenden schlauchförmigen Fortsatz 4 aufweist. Sowohl der schlauchförmige Katheterteil 2 als auch der Ansatz 3 und der Fortsatz 4 bestehen aus einem flexiblen, pharmakologisch unbedenklichen Kunststoff, beispielsweise einem Polyäthylen, Polypropylen, Weich-PVC oder einem entsprechenden Polyurethan. Innerhalb des schlauchförmigen Katheterteiles 2 ist ein Kanal 5 angeordnet, der an den Kanal 5, der den kegelförmigen Ansatz 3 und den schlauchförmigen Fortsatz 4 durchdringt, anschließt. In dem vom kegelförmigen Ansatz 3 abgewendeten Endbereich des Fortsatzes 4 ist der Kanal 5 zur Aufnahme eines Ansatzstückes 6 einer Spritze 7 mit einem sich in Richtung der Spritze erweiternden Luer-Konus 8 versehen. Dies ermöglicht ein direktes Ansetzen der genormten Einmal-Spritzen an dem Katheter 1. Des weiteren ist in dem dem Fortsatz 4 zugewandten Ende des kegelförmigen Ansatzes 3 ein kreisringförmiger Wulst 9, der auch als Kegelabschnitt ausgebildet sein kann, angeordnet.

Im Bereich des vom kegelförmigen Ansatz 3 abgewendeten Endes des schlauchförmigen Katheterteiles 2 sind Ausströmöffnungen 10,11 angeordnet, die radial bzw. senkrecht zu einer Längsrichtung - Längsachse 12 des Kanales 5 - verlaufen. Wie weiters aus dieser Darstellung ersichtlich, endet der Kanal 5 kurz vor dem Ende des schlauchförmigen Katheterteiles 2 und das vom kegelförmigen Ansatz 3 abgewendete Ende - Endbereich 13 - ist abgerundet bzw. kugelkalottenförmig ausgebildet.

Wie aus Fig.2 besser zu ersehen ist, ist die Ausströmöffnung 10 mit einem relativ großen Querschnitt, und zwar rechteckförmig ausgebildet. Eine kürzere Seitenlänge 14 der Ausströmöffnung 10 entspricht in etwa einem Durchmesser 15 des Kanales 5.

In Fig.3 ist die der Ausströmöffnung 10 gegenüberliegend angeordnete Ausströmöffnung 11 gezeigt, die beispielsweise einen runden Querschnitt und außerdem einen geringeren Ausströmquerschnitt als die Ausströmöffnung 10 aufweist. Selbstverständlich können die Ausströmöffnungen 10,11 einen beliebigen Querschnitt haben und außerdem ist es möglich, daß sie in unterschiedlichen radialen Richtungen, beispielsweise in einem Winkel von 30° oder 60° versetzt, den Schlauchmantel des schlauchförmigen Katheterteiles 2 durchdringen.

Wie in der Fig.3 weiters rein schematisch gezeigt ist, ist es auch möglich, den Fortsatz 4 im kegelförmigen Ansatz 3 lösbar zu lagern. Beispielsweise ist es möglich, vor dem Einsetzen des Katheters den Fortsatz 4 mittels eines Gewindes 16 in den kegelförmigen Ansatz 3 einzuschrauben. Gleichermaßen ist es natürlich aber auch möglich, jede beliebige andere Schnapp-Verbindung oder QuetschringVerbindung oder dgl. zu verwenden, um eine dichte Kupplung des Schlauches mit dem kegelförmigen Ansatz 3 zu ermöglichen.

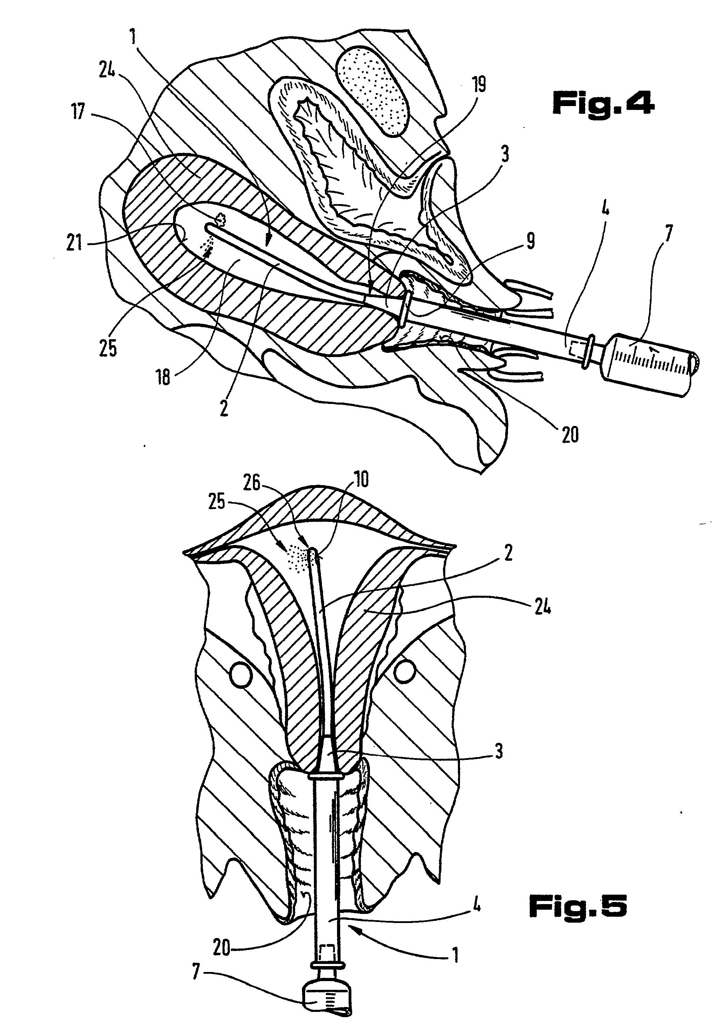

In Fig.4 ist der Katheter 1 in seiner Gebrauchslage zum Einführen von Samen 17 aus der Spritze 7 in das Uteruskavum 18 gezeigt.

Wie ersichtlich, liegt der kegelförmige Ansatz 3 mit dem kreisringförmigen Wulst 9 am Eingang des Zervixkanals 19 an und ergibt eine gute Abdichtung des Uteruskavums 18 sowie eine gute Abstützung des durch die Scheide 20 verlaufenden Fortsatzes 4. Damit wird verhindert, daß während des Einspritzens des Samens 17 mit der Spritze 7 der schlauchförmige Katheterteil 2 des Katheters 1 zu weit in das Uteruskavum 18 eingeschoben wird und die Schleimhaut 21 irritiert bzw. verletzt. Wird nämlich die Schleimhaut 21 verletzt, kommt es zu Blutungen im Uteruskavum, wodurch der eingebrachte Samen zerstört und eine Befruchtung verhindert wird.

Aus dieser Darstellung ist auch besser ersichtlich, welche Vorteile unterschiedliche Durchmesser 22 und 23 des schlauchförmigen Katheterteiles 2 und des Fortsatzes 4 bringen. Durch den größeren Durchmesser 23 des Fortsatzes 4 ist der Katheter 1 im Bereich der Scheide 20 steifer, sodaß ein Einführen des flexibleren schlauchförmigen Katheterteiles 2 durch den Zervixkanal 19 in das Uteruskavum 18 begünstigt wird. Andererseits besteht aber durch die hohe Flexibilität des schlauchförmigen Katheterteiles 2 kaum die Gefahr, daß die Schleimhaut 21 im Uteruskavum 18 oder im Bereich des Zervixkanals 19 verletzt wird.

In Fig.5 ist ebenfalls die Lage des eingebrachten Katheters 1 in der Scheide bei einer Frontalansicht der Gebärmutter 24 zu ersehen. Anstelle der Ausströmöffnung 11 kann eine Luft 25 aus dem Samen 17 in einem der Ausströmöffnung 10 gegenüberliegenden Bereich 26, der luftdurchlässig ausgebildet sein kann, abgeführt werden, wie dies schematisch in Fig.5 gezeigt ist.

Wie anhand der Darstellung in Fig.4 und 5 gezeigt ist, ist es somit möglich, die Spritze 7 zum Einbringen des Samens 17 durch den Katheter 1 außerhalb der Scheide 20 anzuordnen und es kann somit völlig steril gearbeitet werden. Wie weiters in Fig.4 schematisch angedeutet ist, schafft die Anordnung der in den Fig.1 bis 3 und 5 gezeigten beiden Ausströmöffnungen 10 und 11 bzw. des luftdurchlässigen Bereiches 26 die Möglichkeit, daß in Luftblasen des Samens enthaltene verdichtete Luft 25 durch die als Entlastungsbohrung wirkende Ausströmöffnung 11 bzw. den luftdurchlässigen Bereich 26 mit niederem Druck entweichen kann, wodurch die Verletzungsgefahr der Schleimhaut 21 zusätzlich verringert wird.

**Patentansprüche**

1. Katheter (1) zur künstlichen Befruchtung mit einem in die Gebärmutter (24) bzw. durch den inneren Muttermund in das Uteruskavum (18) einführbaren flexiblen schlauchförmigen Katheterteil (2), der in einem, einem Zervixkanal (19) zugeordneten Endbereich einen sich daran anschließenden flexiblen schlauchförmigen Fortsatz (4) aufweist, dessen Länge im wesentlichen der Länge einer Scheide (20) entspricht und im anderen Endbereich stirnseitig verschlossen und abgerundet bzw. kugelkalottenförmig ausgebildet und mit einem Kanal (5), der vor dem abgerundeten Endbereich mit radial angeordneten Ausströmöffnungen (10, 11) verbunden ist, wobei zwischen dem Katheterteil (2) und dem Fortsatz (4) ein sich in Richtung des Fortsatzes (4) vergrößernder kegelförmiger Ansatz (3) angeordnet ist, dadurch gekennzeichnet, daß der Kanal (5) des schlauchförmigen Katheterteiles (2) in dem den kegelförmigen Ansatz (3) gegenüberliegenden Endbereich (13) mit nur zwei in Umfangsrichtung versetzten, radial verlaufenden Ausströmöffnungen (10, 11) versehen ist, von welchen die eine Ausströmöffnung (10) mit einem großen Querschnitt und die weitere Ausströmöffnung (11) mit einem geringeren Ausströmquerschnitt als die eine Ausströmöffnung (10) ausgebildet oder durch einen luftdurchlässigen, insbesondere der einen Ausströmöffnung (10) gegenüberliegenden Bereich im Schlauchmantel des schlauchförmigen Katheterteiles (2) gebildet ist.

2. Katheter nach Anspruch 1, dadurch gekennzeichnet, daß der Kanal (5) des schlauchförmigen Fortsatzes (4) in dem vom kegelförmigen Ansatz (3) abgewendeten Endbereich – wie an sich bekannt – konusförmig erweitert, insbesondere als Luer-Konus (8) ausgebildet ist,

3. Katheter nach Anspruch 1 oder 2, dadurch gekennzeichnet, daß zwischen dem kegelförmigen Ansatz (3) und dem schlauchförmigen Fortsatz (4) ein coaxial zur Bohrung verlaufender kreisringförmiger, insbesondere kegelstumpfartiger Wulst (g) angeordnet ist.

4. Katheter nach einem der Ansprüche 1 bis 3, dadurch gekennzeichnet, daß der Fortsatz (4) und der schlauchförmige Katheterteil (2) über eine Verbindungsvorrichtung lösbar verbunden sind.

5. Katheter nach einem der Ansprüche 1 bis 4, dadurch gekennzeichnet, daß der Katheter (1) einstückig ausgebildet und insbesondere aus einem elastischen Kunststoff, z.B. Polyäthylen bzw. Polypropylen oder Weich-PVC besteht.

6. Katheter nach einem der Ansprüche 1 bis 5, dadurch gekennzeichnet, daß die eine Ausströmöffnung (10) im wesentlichen rechteckförmig ausgebildet ist und eine kürzere Seitenlänge (14) derselben im wesentlichen einem Durchmesser (15) des Kanales (5) entspricht.

7. Katheter nach einem der Ansprüche 1 bis 6, dadurch gekennzeichnet, daß die weitere Ausströmöffnung (11) der einen Ausströmöffnung (10) spiegelbildlich gegenüberliegt.

8. Katheter nach einem der Ansprüche 1 bis 7, dadurch gekennzeichnet, daß der Durchmesser (22) des schlauchförmigen Katheterteiles (2) in etwa halb so groß ist wie der Durchmesser (23) des schlauchförmigen Fortsatzes (4).

**Claims**

1. A catheter (1) for artificial fertilisation, comprising a flexible hose-like catheter section (2) insertible into the uterine cavity (18) of a womb (24) via the inner cervical opening (19), which is provided in a terminal area allocated to the cervical passage with a flexible hose-like extension (4) its length substantially corresponds to the length of a vagina (20) and is closed off and made in rounded or spherical cap form in a other terminal portion, and is connected by a passage (5) to radially extending outflow opening (10, 11) situated before the rounded-off terminal portion, wherein a conical attachment (3) widening rearwardly towards the extension (4) is arranged between the catheter section (2) and the extension (4) characterised in that the passage (5) of the hose-like catheter section (2) in a terminal section (13) facing the conical attachment (3) is provided with only two peripherally staggered and radially extending outflow openings (10, 11), in which one outflow opening (10) is provided with a large outflow cross-section and a further outflow opening (11) has a smaller outflow cross-section as the one outflow opening (10) or is formed by an air-permeable portion situated in the hose jacket of the hose-like catheter section (2) opposite in particular to the one outflow opening (10).

2. A catheter according to claim 1, in which the passage (5) of the hose-like extension (4) is conically widened and in particular is constructed as a Luer taper (8) in a rearward terminal portion facing away from the conical attachment (3).

3. A catheter according to claim 1 or 2, in which an annular and in particular frustoconical enlargement rim (9) extending coaxially with respect to the bore is situated between the conical attachment (3) and the hose-like extension (4).

4. A catheter according to one of the claims 1–3, in which the extension (4) and the hose-like catheter section (2) are releasably coupled via a connecting device.

5. A catheter according to one of the claims 1–4, in which the catheter (1) is constructed in one piece and in particular consists of an elastic plastics material selected from the group comprising polyethylene, polypropylene, soft PVC, and polyurethane.

6. A catheter according to one of the claims 1–5, in which the one outflow opening (10) is made substantially rectangular and that a shorter side length (14) thereof corresponds substantially to a diameter (15) of the passage (5).

7. A catheter according to one of the claims 1–6, in which the further outflow opening (11) is oppositely situated in mirror symmetry to the one outflow opening (10).

8. A catheter according to claims 1–7, in which the diameter (22) of the hose-like catheter section (2) is approximately half as great as the diameter (23) of the hose-like extension (4).

**Revendications**

1. Cathéter (1) pour l'insémination artificielle avec une partie de cathéter (2) de forme tubulaire flexible pouvant être introduite dans la matrice (24) ou, respectivement, par le col interne de l'utérus, dans la cavité utérine (18), qui présente dans une zone extrême affectée au canal cervical (19), un prolongement (4) tubulaire flexible qui s'y raccorde, dont la longueur correspond en principe à la longueur d'un vagin (20) et à son autre zone extrême côté frontal, est obturée et configurée de manière arrondie ou respectivement en forme de calotte sphérique et avec un canal (5) qui est, relié avant la zone extrême arrondie, à des ouvertures agencées radialement d'évacuation (10, 11), et entre la partie de cathéter (2) et le prolongement (4) est agencée une saillie (3) de forme conique s'agrandissant dans la direction du prolongement (4), caractérisé en ce que le canal (5) de la partie de cathéter (2) de forme tutubulaire est pourvu, dans la zone extrême (13) se trouvant face à la saillie conique (3), de deux ouvertures radiales d'évacuation (10, 11) seulement, qui sont décalées en direction périphérique, dont une ouverture d'évacuation (10) est configurée avec une plus grande section tranversale et l'autre ouverture d'évacuation (11) avec une moindre section transversale d'évacuation que celle de l'ouverture d'évacuation (10) ou bien est formé d'une zone perméable à l'air face en particulier à la première ouverture d'évacuation (10) dans l'enveloppe tubulaire de la partie de cathéter (2) de forme tubulaire.

2. Cathéter selon la revendication 1, caractérisé en ce que le canal (5) du prolongement tubulaire (4) est configuré dans la zone extrême opposée à la saillie conique (3), comme on le sait, de façon à s'élargir en forme de cône, en particulier en forme de cône de ruer (8).

3. Cathéter selon la revendication 1 ou 2, caractérisé en ce qu'entre la saillie conique (3) et le prolongement tubulaire (4) est agencé un bourrelet annulaire (9), en particulier en forme de tronc de cône passant coaxialement au perçage.

4. Cathéter selon l'une quelconque des revendications 1 à 3, caractérisé en ce que le prolongement (4) et la partie de cathéter (2) de forme tubulaire sont reliés de manière détachable par un dispositif de liaison.

5. Cathéter selon l'une quelconque des revendications 1 à 4, caractérisé en ce que le cathéter (1) est formé en une pièce et se compose en particulier d'une matière élastique synthétique, par exemple, du polyéthylène ou du polypropylène ou bien du PVC souple.

6. Cathéter selon l'une quelconque des revendications 1 à 5, caractérisé en ce qu'une ouverture d'évacuation (10) est en principe de configuration rectangulaire et son côté court (14) correspond en principe au diamètre (15) du canal (5).

7. Cathéter selon l'une quelconque des revendications 1 à 6, caractérisé en ce que l'autre ouverture d'évacuation (11) se trouve face à la première ouverture d'évacuation (10) en image miroir.

8. Cathéter selon l'une quelconque des revendications 1 à 7, caractérisé en ce que le diamètre (22) de la partie tubulaire du cathéter (2) est à peu près égal à la moitié du diamètre (23) du prolongement tubulaire (4).

**Fig.1**

**Fig.2**

**Fig.3**

**Fig.4**

**Fig.5**